# EUROPÄISCHE PATENTSCHRIFT

(11) **EP 0 385 114 B1**
(45) Veröffentlichungstag und Bekanntmachung des Hinweises auf die Patenterteilung: **03.03.1993**
(21) Anmeldenummer: 90101761.6
(22) Anmeldetag: 30.01.1990
(51) Int. Cl.: A61F 5/01

(54) **Cervicalstütze**
Cervical collar
Collier cervical

(30) Priorität: 28.02.1989 DE 3906232
(43) Veröffentlichungstag der Anmeldung: 05.09.1990
(73) Patentinhaber: ADEV, GESELLSCHAFT FÜR ENTWICKLUNG UND VERTRIEB VON MEDIZINTECHNISCHEN ARTIKELN MBH, D-23556 Lübeck (DE)
(72) Erfinder: Grundei, Jaana, D-2400 Lübeck (DE); Timmermann, Andreas, D-2360 Klein Rönnau (DE)
(74) Vertreter: Weiss, Christian, Dipl.-Ing.

(56) Entgegenhaltungen:
- EP-A- 0 301 368
- WO-A-87/01028
- DE-A- 2 404 683
- US-A- 3 756 226
- US-A- 3 916 885

## Beschreibung

Die Erfindung betrifft eine Cervicalstütze, bestehend aus einem im wesentlichen formstabilen, gegebenenfalls mit einem Überzug aus Textilmaterial versehenen und um den Hals des Patienten legbaren Stützkörper aus elastischem Schaumstoff, dessen freie Enden sich hinten im Nackenstützbereich überlappen und lösbar miteinander verbindbar sind, während die beiden seitlichen Kieferstützbereiche hinsichtlich ihrer oberen und unteren Randkonturen der anatomischen Form des Kinns bzw. halsnahen Körperbereichs angepaßt sind.

Eine mit diesen Gestaltungsmerkmalen ausgeführte Cervicalstütze ist aus der DE-A-24 04 683 zu entnehmen. Diese bekannte Cervicalstütze hat sich gut bewährt, doch ergeben sich in der Praxis je nach Indikation unterschiedliche Anforderungen an die Stützfunktion, denen allenfalls durch unterschiedliche Ausführung individuell Rechnung getragen werden kann, wobei solche Ausführungsmerkmale jedoch wiederum unveränderlich sind.

Die hier vorliegende mangelnde Anpaßbarkeit an die indikationsbedingten Erfordernisse kann auch nicht durch die in dem DE-U-70 46 248 beschriebene Cervicalstütze überwunden werden, bei der es sich um eine an die Hals- und Schulterpartie angepaßte und durch einen senkrecht angeordneten lösbaren Verschluß geteilte Cervicalstütze handelt. Diese kann aus Leder bestehen, aber auch aus einem Textilmaterial, wobei dann eingearbeitete und/oder herausnehmbare Versteifungsorgane vorgesehen sind, die für die dem Textmaterial fehlende Steifigkeit sorgen.

Ein therapeutischer Halskragen ist bekannt geworden aus der W0-Al-87/01028. Dieser besteht aus einem Formteil, welches mittels im hinteren Halsbereich oder alternativ dazu mittels eines es teilweise umschließendes Versteifungsteils partiell versteift ist. Die Versteifungsteile sind beispielsweise in Textiltaschen in textilen Überzugsgewebe eingelassen. Die Versteifungsteile üben eine vergleichbare Funktion aus wie die Versteifungsorgane gemäß der vorerwähnten Druckschrift.

Es ist die Aufgabe der Erfindung, eine Cervicalstütze zu schaffen, die entsprechend den Erfordernissen der jeweiligen Indikation herrichtbar und an im Verlaufe der Therapie sich ergebende veränderte Erfordernisse leicht anpaßbar ist.

Diese Aufgabe wird erfindungsgemäß dadurch gelöst, daß der Stützkörper mit mindestens einem radialen Durchbruch zur Aufnahme eines darin für einen Austausch lösbar befestigbaren, den Stützkörper partiell stabilisierenden Stützelementes versehen ist, das einen in den Durchbruch einsteckbaren und dabei den Stützkörper durchgreifenden Schaft sowie einen großflächigen, am Innenumfang des Stützkörpers zur Anlage kommenden und diesen dort versteifenden Kopf aufweist.

Die mit dieser Ausführung erzielbaren Vorteile stehen insbesondere in der Möglichkeit, mit einer Cervicalstütze unterschiedliche Krankheitsbilder zu berücksichtigen. So kann beispielweise durch Verzicht auf jegliche Stützelemente die Eignung für eine leichte Ruhestellung bei relativ geringer Stützfunktion, d.h. im wesentlichen Konzentration auf eine Wärmungsfunktion am Ende einer Behandlung erreicht werden. Es kann aber auch eine Einstellung für eine Ruhigstellung mit starker Stützfunktion vorgenommen werden oder auch eine starke Ruhigstellung mit weitgehend versteifter Stütze vorgesehen werden.

Die die partielle Versteifung des Stützkörpers regulierenden Stützelemente sind in dem Stützkörper lösbar befestigbar. Zu diesem Zweck kann vorgesehen sein, daß der Schaft jedes Stützelementes in montiertem Zustand ein nach außen über den Umfang des Stützkörpers hinaus vorstehendes Ende aufweist, welches in einem an der Außenfläche des Stützkörpers anliegenden und diesen teilweise umspannenden flexiblen Band lösbar verankerbar ist. Diese lösbare Verankerung kann dadurch erreicht werden, daß das Ende des Schaftes pilzartig verdickt ausgeführt ist und das Band je einen dem Querschnitt des Schaftes entsprechenden Durchbruch aufweist, so daß das pilzartig verdickte Ende in diese Durchbrüche eingesprengt werden kann.

Die Erfindung wird nachstehend anhand eines in der Zeichnung dargestellten Ausführugnsbeispiels näher erläutert.Es zeigen:
Figur 1 eine Cervicalstütze mit drei Stützelementen in Draufsicht,
Figur 2 eine Cervicalstütze gemäß Figur 1 mit einem dieselbe zusätzlich versteifenden Band am äußeren Umfang mit in diesem verankerten Stützelement,
Figur 3 eine Seitenansicht der Cervicalstütze aus Figur 1,
Figur 4 eine Seitenansicht der Cervicalstütze aus Figur 2.

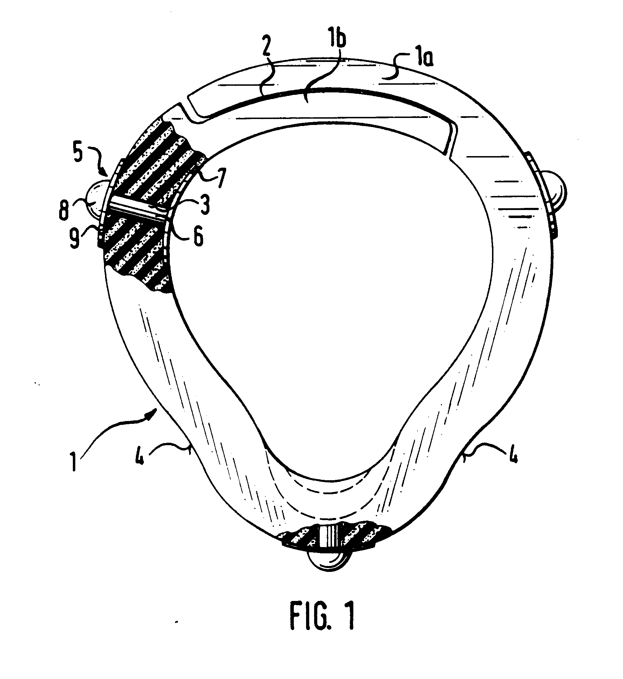

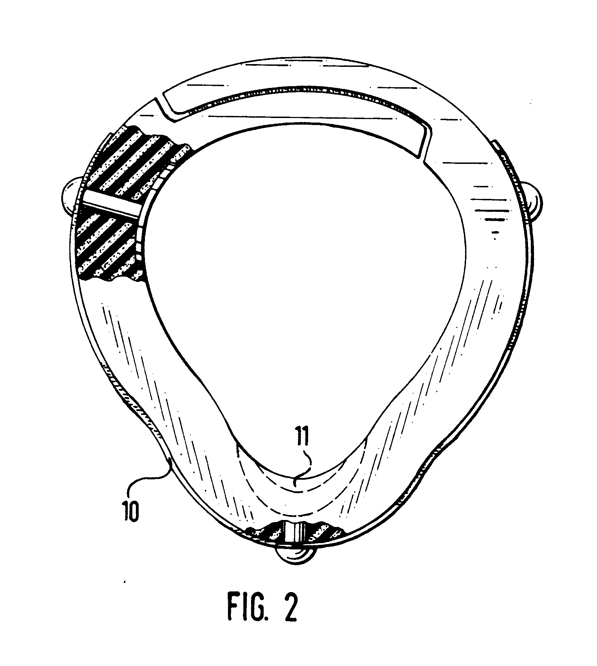

Die aus einem flexiblen und formstabilen Stützkörper 1 aus elastischem Schaumstoff bestehende Cervicalstütze wird um den Hals eines Patienten gelegt und schmiegt sich den anatomischen Gegebenheiten an. Dabei wird sie im Nackenbereich an sich überlappenden Endteilen 1a, 1b (Figuren 1 und 2) mittels eines Verschlusses, vorzugsweise mittels eines sogenannten Klettverschlusses 2 zusammengehalten. Die Formstabilität des Stützkörpers 1 ist derart, daß dieser, auch wenn nicht an dem Hals des Patienten angelegt, umfangmäßig einen formstabilen Körper bildet, der zum Anlegen und Abnehmen aufgrund seiner Flexibilität im Nackenbereich auseinandergespreizt werden kann, und der sich aufgrund der dem Material innewohnende Rückstellkräfte selbst wieder zu schließen vermag.

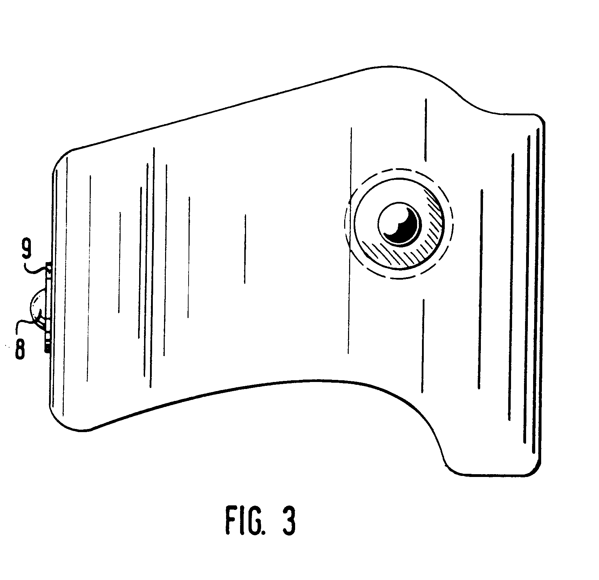

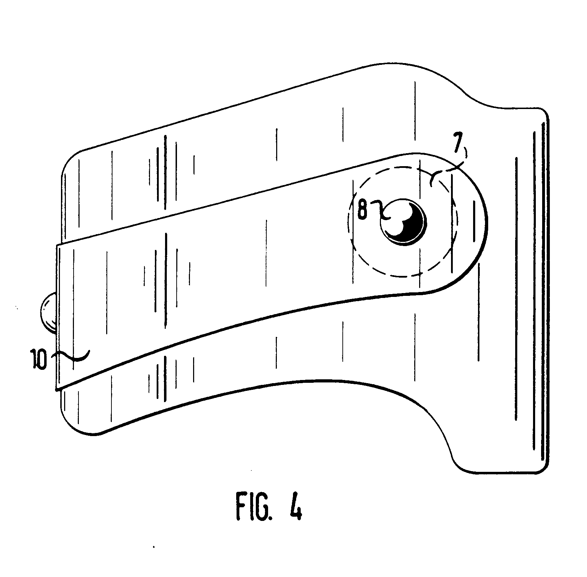

Der Stützkörper 1 ist an seinem oberen und an seinem unteren Umfangsrand im wesentlichen den anatomischen Gegebenheiten des an den Hals angrenzenden Kopf- und Schulterbereichs des Patienten angepaßt. Diese Gestaltung ist am besten aus den Figuren 3 und 4 zu erkennen. Der Stützkörper 1 wird von, in dem dargestellten Ausführungsbeispiel drei, Durchbrüchen 3 radial durchdrungen. Weitere Durchbrüche 3 können beispielsweise auch im Bereich der konkaven Einbuchtungen 4 in der äußeren Umfangsfläche angeordnet sein. Die Durchbrüche 3 dienen der Aufnahme von Stützelementen 5.

Jedes der Stützelemente 5 weist einen Schaft 6 auf, dessen eines Ende mit einem großflächigen Kopf 7 von im wesentlichen scheibenförmiger Gestalt versehen ist, und dessen anderes Ende 8 pilzartig verdickt ausgeführt ist. Das Stützelement 5 besteht vorzugsweise aus einem flexiblen Kunststoffmaterial halbsteifen Charakters. Das pilzartig verdickte Ende 8 des Stützelementes 5 ist wahlweise in die Mittenbohrung einer Scheibe 9 eindrückbar, die ebenfalls aus dem beschriebenen Material bestehen kann. Die Funktion der Scheibe 9 kann auch ein flexibles Band 10 übernehmen, das den Stützkörper 1 an dessen Außenfläche anliegend unter Aussparung des Bereichs der überlappenden Enden 1a und Ib umspannt und mit Bohrungen versehen ist, die mit der Position der Durchbrüche 3 übereinstimmen und der Aufnahme des Endes 8 der Stützelemente dienen.

Die Länge des Schaftes 6 der Stützelemente 5 ist so bemessen, daß der Kopf 7 derselben nach dem Fixieren durch Eindrücken des pilzartig verdickten Endes 8 in eine Scheibe 9 oder das Band 10 außenbündig mit der Innenkontur des Stützkörpers 1 ist. Im Bereich der in der Nähe des Kehlkopfes zu liegen kommenden Partie ist der Stützkörper 1 mit einer Einsenkung 11 an dessen Innenkontur versehen, so daß der Kopf 7 eines dort angeordneten Stützelementes 5 versenkt liegt, womit ein Freiraum für den Kehlkopf des Patienten gebildet wird.

Die erfindungsgemäße Cervicalstütze wird nach der Diagnose des jeweiligen Krankheitsbildes und den daraus hergeleiteten Erfordernissen der Indikation hergerichtet, in dem durch Einsetzen von Stützelementen 5 die jeweils für therapeutisch hilfreich erachtete örtliche Steifigkeit des Stützkörpers 1 vorgesehen wird. Dies kann je nach vorliegenden Bedürfnissen unter fixieren der Stützelemente 5 mit Scheiben 9 oder durch das Band 10 geschehen, wobei die letztere Ausführung für die größtmögliche Steifigkeit sorgt.

Nach Herrichten der Cervicalstütze in dieser Weise wird diese in einen hautverträglichen textilen Überzug gekleidet, der im Bereich der überlappenden Enden 1a und 1b mit dem Klettverschluß 2 ausgestattet sein kann.

Im Verlaufe der Therapie kann nun je nach fortschritt des Heilungsprozesses durch Entnahme einzelner Stützelemente 5 oder Ersatz des Bandes 10 durch Scheiben 9 eine schrittweise Reduzierung der Stützfunktion vorgenommen werden.

## Patentansprüche

1. Cervicalstütze, bestehend aus einem im wesentlichen formstabilen, gegebenenfalls mit einem Überzug aus Textilmaterial versehenen und um den Hals des Patienten legbaren Stützkörper (1) aus elastischem Schaumstoff, dessen freie Enden sich hinten im Nackenstützbereich überlappen und lösbar miteinander verbindbar sind, während die beiden seitlichen Kieferstützbereiche hinsichtlich ihrer oberen und unteren Randkonturen der anatomischen Form des Kinns bzw. halsnahen Körperbereichs angepaßt sind, dadurch gekennzeichnet, daß der Stützkörper (1) mit mindestens einem radialen Durchbruch (3) zur Aufnahme eines darin für einen Austausch lösbar befestigbaren, den Stützkörper partiell stabilisierenden Stützelementes (5) versehen ist, das einen in den Durchbruch (3) einsteckbaren und dabei den Stützkörper (1) durchgreifenden Schaft (6) sowie einen großflächigen, am Innenumfang des Stützkörpers (1) zur Anlage kommenden und diesen dort versteifenden Kopf (7) aufweist.

2. Cervicalstütze nach Anspruch 1, dadurch gekennzeichnet, daß der Schaft (6) jedes Stützelementes (5) in montiertem Zustand ein nach außen über den Umfang des Stützkörpers (1) hinaus vorstehendes Ende (8) aufweist, welches in einem an der Außenfläche des Stützkörpers (1) anliegenden und diesen teilweise umspannenden flexiblen Band (10) lösbar verankerbar ist.

3. Cervicalstütze nach Anspruch 1 oder 2, dadurch gekennzeichnet, daß das Ende (8) des Schaftes (6) pilzartig verdickt ausgeführt ist und das Band (10) je einen dem Querschnitt des Schaftes (6) entsprechenden Durchbruch aufweist.

## Claims

1. A cervical collar comprising a substantially inherently stable support body (1) of elastic foam material which, if necessary, can encircle the neck of a patient, the free end parts of the support body overlapping each other in the nape support rear region and being releasably connectable to each other whilst, as regards their upper and lower edge contours, the two latteral jaw support regions are adapted to the anatomical shape of the chin or to the parts of the body adjacent to the neck, characterized in that the support body (1) is provided with at least one radial opening (3) to adopt a support member (5) partially stabilizing the support body, the purpose of replacement being releasably connectable for, the support member (5) comprising a shank (6) being insertable into the opening (3) and extending through the support body (1) as well as a large area head (7) for the abutment against the inner circumference of the support body (1) and for locally stiffening the support body (1).

2. A cervical collar according to claim 1, characterized in that, being mounted, the shank (6) of each support member (5) comprises an end (8) projecting outwardly beyond the circumference of the support body (1), the said end being releasably attached to a flexible band (10) abutting the outer surface of the support body (1) and partially embracing it.

3. A cervical collar according to claims 1 or 2, characterized in that the end (8) of the shank (6) has a thickened mushroom-head configuration and in that the band (10) comprises an opening that corresponds in each case to the cross-section of the shank (6).

## Revendications

1. Minerve, constituée d'un corps d'appui (1), sensiblement indéformable, pourvu, le cas échéant, d'un revêtement textile et à appliquer autour du cou du patient, en mousse élastique, dont les extrémités libres se recouvrent dans la zone d'appui du cou et peuvent être assemblées entre elles de manière amovible, tandis que les deux zones latérales d'appui du maxillaire sont adaptées, en ce qui concerne leurs contours périphériques supérieurs et inférieurs, à la forme anatomique du menton ou de la zone du corps voisine du cou, caractérisée en ce que le corps d'appui (1) est pourvu d'au moins un ajour (3) radial destiné à loger un élément d'appui (5), à fixer de manière amovible, à l'intérieur de celui-ci, pour un échange, stabilisant partiellement le corps d'appui, lequel élément d'appui comporte une tige (6) à insérer dans l'ajour (3) et traversant ainsi le corps d'appui (1), ainsi qu'une tête (7) de grande surface, venant s'appliquer contre le pourtour intérieur du corps d'appui (1) et raidissant celui-ci à cet endroit.

2. Minerve selon la revendication 1, caractérisée en ce que la tige (6) de chaque élément d'appui (5) comporte, à l'état monté, une extrémité (8) dépassant vers l'extérieur du pourtour du corps d'appui (1), laquelle extrémité peut être ancrée de manière amovible dans une bande (10) flexible s'appliquant contre la surface extérieure du corps d'appui (1) et entourant celui-ci partiellement, en le serrant.

3. Minerve selon la revendication 1 ou 2, caractérisée en ce que l'extrémité (8) de la tige (6) est surépaissie en forme de champignon et en ce que la bande (10) présente un ajour correspondant à la section transversale de la tige (6).
